# EUROPEAN PATENT APPLICATION

(11) **EP 3 002 336 A1**
(43) Date of publication of application: **06.04.2016**
(21) Application number: 14306562.1
(22) Date of filing: 03.10.2014
(51) Int. Cl.: C12Q 1/68

(54) **Polymorphisms predictive of pancreatic cancer**

(71) Applicant: Université d'Aix Marseille, 13007 Marseille 7 (FR); Assistance Publique Hôpitaux de Marseille, 13354 Marseille Cedex 5 (FR); INSERM (Institut National de la Santé et de la Recherche Médicale), 75013 Paris (FR)
(72) Inventor: Lombardo, Dominique, 13008 Marseille (FR); Martinez, Emmanuelle, 13002 Marseille (FR); Mas, Eric, 13010 Marseille (FR); Silvy, Françoise, 13005 Marseille (FR); Ouaissi, Mehdi, 13009 Marseille (FR); Fina, Frédéric, 13240 Septèmes les Vallons (FR)
(74) Representative: Pierru, Bénédicte

(57) **Abstract**

The present invention relates to a method for determining a predisposition to, prognosing or diagnosing pancreatic cancer in a subject.

SEQUENCE LISTING

<110>  Université d'Aix-Marseille
       Assistance Publique - Hôpitaux de Marseille
       INSERM

<120>  Polymorphisms predictive of pancreatic cancer

<130>  B1878EP

<160>  13

<170>  PatentIn version 3.5

<210>  1
<211>  2386
<212>  DNA
<213>  Homo sapiens

<400>  1
ggccacccag aggctgatgc tcaccatggg gcgcctgcaa ctggttgtgt tgggcctcac      60

ctgctgctgg gcagtggcga gtgccgcgaa gctgggcgcc gtgtacacag aaggtgggtt     120

cgtggaaggc gtcaataaga agctcggcct cctgggtgac tctgtggaca tcttcaaggg     180

catccccttc gcagctccca ccaaggccct ggaaaatcct cagccacatc ctggctggca     240

agggaccctg aaggccaaga acttcaagaa gagatgcctg caggccacca tcacccagga     300

cagcacctac ggggatgaag actgcctgta cctcaacatt tgggtgcccc agggcaggaa     360

gcaagtctcc cgggacctgc ccgttatgat ctggatctat ggaggcgcct tcctcatggg     420

gtccggccat ggggccaact tcctcaacaa ctacctgtat gacggcgagg agatcgccac     480

acgcggaaac gtcatcgtgg tcaccttcaa ctaccgtgtc ggcccccttg ggttcctcag     540

cactggggac gccaatctgc caggtaacta tggccttcgg gatcagcaca tggccattgc     600

ttgggtgaag aggaatatcg cggccttcgg gggggacccc aacaacatca cgctcttcgg     660

ggagtctgct ggaggtgcca gcgtctctct gcagaccctc tccccctaca acaagggcct     720

catccggcga gccatcagcc agagcggcgt ggccctgagt ccctgggtca tccagaaaaa     780

cccactcttc tgggccaaaa aggtggctga gaaggtgggt tgccctgtgg gtgatgccgc     840

caggatggcc cagtgtctga aggttactga tccccgagcc ctgacgctgg cctataaggt     900

gccgctggca ggcctggagt accccatgct gcactatgtg ggcttcgtcc ctgtcattga     960

tggagacttc atccccgctg acccgatcaa cctgtacgcc aacgccgccg acatcgacta    1020

tatagcaggc accaacaaca tggacggcca catcttcgcc agcatcgaca tgcctgccat    1080

caacaagggc aacaagaaag tcacggagga ggacttctac aagctggtca gtgagttcac    1140

aatcaccaag gggctcagag gcgccaagac gacctttgat gtctacaccg agtcctgggc    1200

ccaggaccca tcccaggaga ataagaagaa gactgtggtg gactttgaga ccgatgtcct    1260

cttcctggtg cccaccgaga ttgccctagc ccagcacaga gccaatgcca agagtgccaa    1320

gacctacgcc tacctgtttt cccatccctc tcggatgccc gtctacccca aatgggtggg 1380

ggccgaccat gcagatgaca ttcagtacgt tttcgggaag cccttcgcca cccccacggg 1440

ctaccggccc caagacagga cagtctctaa ggccatgatc gcctactgga ccaactttgc 1500

caaaacaggg gaccccaaca tgggcgactc ggctgtgccc acacactggg aaccctacac 1560

tacggaaaac agcggctacc tggagatcac caagaagatg ggcagcagct ccatgaagcg 1620

gagcctgaga accaacttcc tgcgctactg gaccctcacc tatctggcgc tgcccacagt 1680

gaccgaccag gaggccaccc ctgtgccccc cacaggggac tccgaggcca ctcccgtgcc 1740

ccccacgggt gactccgaga ccgcccccgt gccgcccacg ggtgactccg ggcccccccc 1800

cgtgccgccc acgggtgact ccggggcccc ccccgtgccg cccacgggtg actccggggc 1860

cccccccgtg ccgcccacgg gtgactccgg ggcccccccc gtgccgccca cgggtgactc 1920

cggggccccc cccgtgccgc ccacgggtga ctccggggcc ccccccgtgc cgcccacggg 1980

tgactccggg gccccccccg tgccgcccac gggtgactcc ggcgcccccc ccgtgccgcc 2040

cacgggtgac gccgggcccc cccccgtgcc gcccacgggt gactccggcg ccccccccgt 2100

gccgcccacg ggtgactccg ggcccccccc cgtgaccccc acgggtgact ccgagaccgc 2160

ccccgtgccg cccacgggtg actccggggc cccccctgtg ccccccacgg gtgactctga 2220

ggctgcccct gtgcccccca cagatgactc caaggaagct cagatgcctg cagtcattag 2280

gttttagcgt cccatgagcc ttggtatcaa gaggccacaa gagtgggacc ccaggggctc 2340

ccctcccatc ttgagctctt cctgaataaa gcctcatacc cctgtc 2386

<210> 2
<211> 756
<212> PRT
<213> Homo sapiens

<400> 2

Met Leu Thr Met Gly Arg Leu Gln Leu Val Val Leu Gly Leu Thr Cys
1               5                   10                  15

Cys Trp Ala Val Ala Ser Ala Ala Lys Leu Gly Ala Val Tyr Thr Glu
            20                  25                  30

Gly Gly Phe Val Glu Gly Val Asn Lys Lys Leu Gly Leu Leu Gly Asp
        35                  40                  45

Ser Val Asp Ile Phe Lys Gly Ile Pro Phe Ala Ala Pro Thr Lys Ala
    50                  55                  60

Leu Glu Asn Pro Gln Pro His Pro Gly Trp Gln Gly Thr Leu Lys Ala
65                  70                  75                  80

Lys Asn Phe Lys Lys Arg Cys Leu Gln Ala Thr Ile Thr Gln Asp Ser
                85                  90                  95

Thr Tyr Gly Asp Glu Asp Cys Leu Tyr Leu Asn Ile Trp Val Pro Gln
            100                 105                 110

Gly Arg Lys Gln Val Ser Arg Asp Leu Pro Val Met Ile Trp Ile Tyr
        115                 120                 125

Gly Gly Ala Phe Leu Met Gly Ser Gly His Gly Ala Asn Phe Leu Asn
    130                 135                 140

Asn Tyr Leu Tyr Asp Gly Glu Glu Ile Ala Thr Arg Gly Asn Val Ile
145                 150                 155                 160

Val Val Thr Phe Asn Tyr Arg Val Gly Pro Leu Gly Phe Leu Ser Thr
                165                 170                 175

Gly Asp Ala Asn Leu Pro Gly Asn Tyr Gly Leu Arg Asp Gln His Met
            180                 185                 190

Ala Ile Ala Trp Val Lys Arg Asn Ile Ala Ala Phe Gly Gly Asp Pro
        195                 200                 205

Asn Asn Ile Thr Leu Phe Gly Glu Ser Ala Gly Gly Ala Ser Val Ser
    210                 215                 220

Leu Gln Thr Leu Ser Pro Tyr Asn Lys Gly Leu Ile Arg Arg Ala Ile
225                 230                 235                 240

Ser Gln Ser Gly Val Ala Leu Ser Pro Trp Val Ile Gln Lys Asn Pro
                245                 250                 255

Leu Phe Trp Ala Lys Lys Val Ala Glu Lys Val Gly Cys Pro Val Gly
            260                 265                 270

Asp Ala Ala Arg Met Ala Gln Cys Leu Lys Val Thr Asp Pro Arg Ala
        275                 280                 285

Leu Thr Leu Ala Tyr Lys Val Pro Leu Ala Gly Leu Glu Tyr Pro Met
    290                 295                 300

Leu His Tyr Val Gly Phe Val Pro Val Ile Asp Gly Asp Phe Ile Pro
305                 310                 315                 320

Ala Asp Pro Ile Asn Leu Tyr Ala Asn Ala Ala Asp Ile Asp Tyr Ile

325                 330                 335

Ala Gly Thr Asn Asn Met Asp Gly His Ile Phe Ala Ser Ile Asp Met
            340                 345                 350

Pro Ala Ile Asn Lys Gly Asn Lys Lys Val Thr Glu Glu Asp Phe Tyr
        355                 360                 365

Lys Leu Val Ser Glu Phe Thr Ile Thr Lys Gly Leu Arg Gly Ala Lys
    370                 375                 380

Thr Thr Phe Asp Val Tyr Thr Glu Ser Trp Ala Gln Asp Pro Ser Gln
385                 390                 395                 400

Glu Asn Lys Lys Lys Thr Val Val Asp Phe Glu Thr Asp Val Leu Phe
                405                 410                 415

Leu Val Pro Thr Glu Ile Ala Leu Ala Gln His Arg Ala Asn Ala Lys
            420                 425                 430

Ser Ala Lys Thr Tyr Ala Tyr Leu Phe Ser His Pro Ser Arg Met Pro
        435                 440                 445

Val Tyr Pro Lys Trp Val Gly Ala Asp His Ala Asp Asp Ile Gln Tyr
    450                 455                 460

Val Phe Gly Lys Pro Phe Ala Thr Pro Thr Gly Tyr Arg Pro Gln Asp
465                 470                 475                 480

Arg Thr Val Ser Lys Ala Met Ile Ala Tyr Trp Thr Asn Phe Ala Lys
                485                 490                 495

Thr Gly Asp Pro Asn Met Gly Asp Ser Ala Val Pro Thr His Trp Glu
            500                 505                 510

Pro Tyr Thr Thr Glu Asn Ser Gly Tyr Leu Glu Ile Thr Lys Lys Met
        515                 520                 525

Gly Ser Ser Ser Met Lys Arg Ser Leu Arg Thr Asn Phe Leu Arg Tyr
    530                 535                 540

Trp Thr Leu Thr Tyr Leu Ala Leu Pro Thr Val Thr Asp Gln Glu Ala
545                 550                 555                 560

Thr Pro Val Pro Pro Thr Gly Asp Ser Glu Ala Thr Pro Val Pro Pro
                565                 570                 575

Thr Gly Asp Ser Glu Thr Ala Pro Val Pro Pro Thr Gly Asp Ser Gly
                580                 585                 590

Ala Pro Pro Val Pro Pro Thr Gly Asp Ser Gly Ala Pro Pro Val Pro
            595                 600                 605

Pro Thr Gly Asp Ser Gly Ala Pro Pro Val Pro Pro Thr Gly Asp Ser
    610                 615                 620

Gly Ala Pro Pro Val Pro Pro Thr Gly Asp Ser Gly Ala Pro Pro Val
625                 630                 635                 640

Pro Pro Thr Gly Asp Ser Gly Ala Pro Pro Val Pro Pro Thr Gly Asp
                645                 650                 655

Ser Gly Ala Pro Pro Val Pro Pro Thr Gly Asp Ser Gly Ala Pro Pro
            660                 665                 670

Val Pro Pro Thr Gly Asp Ala Gly Pro Pro Pro Val Pro Pro Thr Gly
        675                 680                 685

Asp Ser Gly Ala Pro Pro Val Pro Pro Thr Gly Asp Ser Gly Ala Pro
    690                 695                 700

Pro Val Thr Pro Thr Gly Asp Ser Glu Thr Ala Pro Val Pro Pro Thr
705                 710                 715                 720

Gly Asp Ser Gly Ala Pro Pro Val Pro Pro Thr Gly Asp Ser Glu Ala
                725                 730                 735

Ala Pro Val Pro Pro Thr Asp Asp Ser Lys Glu Ala Gln Met Pro Ala
            740                 745                 750

Val Ile Arg Phe
        755

<210>  3
<211>  1001
<212>  DNA
<213>  Homo sapiens

<220>
<221>  allele
<222>  (501)..(501)
<223>  y = c or t

<400>  3
accacgaggc cttgttccct catttgccag tggagggact ttgggcaagt cacttaacct      60

ccccctgcat cggaatccat gtgtgtttga ggatgagagt tactggcaga gccccaagcc     120

catgcacgtg cacagccagt gcccagtatg cagtgagggg catggtgccc agggccagct 180

cagagggcgg ggatggctca ggcgtgcagg tggagagcag ggcttcagcc ccctgggagt 240

ccccagcccc tgcacagcct cttctcactc tgcagggacc ccaacatggg cgactcggct 300

gtgcccacac actgggaacc ctacactacg gaaaacagcg gctacctgga gatcaccaag 360

aagatgggca gcagctccat gaagcggagc ctgagaacca acttcctgcg ctactggacc 420

ctcacctatc tggcgctgcc cacagtgacc gaccaggagg ccacccctgt gccccccaca 480

ggggactccg aggccactcc ygtgcccccc acgggtgact ccgagaccgc ccccgtgccg 540

cccacgggtg actccggggc cccccccgtg ccgcccacgg gtgactccgg ggcccccccc 600

gtgccgccca cgggtgactc cggggccccc cccgtgccgc ccacgggtga ctccggggcc 660

ccccccgtgc cgcccacggg tgactccggg gccccccccg tgccgcccac gggtgactcc 720

ggggcccccc ccgtgccgcc cacgggtgac tccggggccc cccccgtgcc gcccacgggt 780

gactccggcg ccccccccgt gccgcccacg ggtgacgccg ggcccccccc cgtgccgccc 840

acgggtgact ccggcgcccc ccccgtgccg cccacgggtg actccggggc cccccccgtg 900

acccccacgg gtgactccga gaccgccccc gtgccgccca cgggtgactc cggggccccc 960

cctgtgcccc ccacgggtga ctctgaggct gcccctgtgc c 1001

<210> 4
<211> 20
<212> DNA
<213> Artificial Sequence

<220>
<223> sense primer exon 11 of the BSDL gene

<400> 4
gaaccaactt cctgcgctac 20

<210> 5
<211> 21
<212> DNA
<213> Artificial Sequence

<220>
<223> sense primer exon 11 of the BSDL gene

<400> 5
tcttctcact ctgcagggac c 21

<210> 6
<211> 22
<212> DNA
<213> Artificial Sequence

<220>
<223> antisense primer exon 11 of the BSDL gene

<400>  6
caggggtatg aggctttatt ca                                                22


<210>  7
<211>  1203
<212>  DNA
<213>  Homo sapiens

<400>  7
gtaagacgtg ggttgagtgc agggcggagg gccacagccg agaagggcct cccaccacga       60

ggccttgttc cctcatttgc cagtggaggg actttgggca agtcacttaa cctccccctg      120

catcggaatc catgtgtgtt tgaggatgag agttactggc agagccccaa gcccatgcac      180

gtgcacagcc agtgcccagt atgcagtgag gggcatggtg cccagggcca gctcagaggg      240

cggggatggc tcaggcgtgc aggtggagag cagggcttca gccccctggg agtccccagc      300

ccctgcacag cctcttctca ctctgcaggg accccaacat gggcgactcg gctgtgccca      360

cacactggga accctacact acggaaaaca gcggctacct ggagatcacc aagaagatgg      420

gcagcagctc catgaagcgg agcctgagaa ccaacttcct gcgctactgg accctcacct      480

atctggcgct gcccacaggg accgaccagg aggccacccc tgtgcccccc acaggggact      540

ccgaggccac tcccgtgacc cccacgggtg actccgagac cgcccccgtg ccgcccacgg      600

gcgactccgg ggcccccccc gtgccgccca cgggtgactc cggggccccc cccgtgccgc      660

ccacgggtga ctccggggcc ccccccgtgc cgcccacggg tgactccggg gccccccccg      720

tgccgcccac gggtgactcc ggggcccccc ccgtgccgcc cacgggtgac tccggggccc      780

cccccgtgcc gcccacgggt gactccgggg ccccccccgt gccgcccacg ggtgactccg      840

gcgccccccc cgtgccgccc acgggtgacg ccgggccccc ccccgtgccg cccacgggtg      900

actccggcgc cccccccgtg ccgcccacgg gtgactccgg ggcccccccc gtgaccccca      960

cgggtgactc cgagaccgcc cccgtgccgc ccacgggtga ctccggggcc ccccctgtgc     1020

cccccacggg tgactctgag gctgcccctg tgccccccac agatgactcc aaggaagctc     1080

agatgcctgc agtcattagg ttttagcgtc ccatgagcct tggtatcaag aggccacaag     1140

agtgggaccc caggggctcc cctcccatct tgagctcttc ctgaataaag cctcataccc     1200

ctg                                                                    1203


<210>  8
<211>  14
<212>  DNA
<213>  Artificial Sequence

<220>
<223>  Nucleic acid probe specific to the C allele at the polymorphism
       site rs488087

<400>  8
   tggggggcac ggga                                                          14


<210>  9
<211>  14
<212>  DNA
<213>  Artificial Sequence

<220>
<223>  nucleic acid probe specific to the T allele at the polymorphism
       site rs488087

<400>  9
   tggggggcac agga                                                          14


<210>  10
<211>  14
<212>  DNA
<213>  Artificial Sequence

<220>
<223>  Forward primer to amplify the region of exon 11 of the BSDL gene
       comprising the polymorphism site rs 488087

<400>  10
cccccacagg ggac                                                         14


<210>  11
<211>  15
<212>  DNA
<213>  Artificial Sequence

<220>
<223>  Reverse primer to amplify the region of exon 11 of the BSDL gene
       comprising the polymorphism site rs 488087

<400>  11
cggtctcgga gtcac                                                        15


<210>  12
<211>  14
<212>  DNA
<213>  Artificial Sequence

<220>
<223>  nucleic acid probe specific to the C allele at the polymorphism
       site rs488087 attached to a fluorophore (HEX) and a quencher
       (BHQ1)


<220>
<221>  misc_feature
<223>  5'- hexachloro-6-carboxyfluorescein is attached to the 5' end and
       BHQ1 quencher is attached to the 3' end

<400>  12
   tggggggcac ggga                                                          14

<210> 13
<211> 14
<212> DNA
<213> Artificial Sequence

<220>
<223> nucleic probe specific to the T allele at the polymorphism site
      rs488087 comprising a fluorophore (6-FAM) and a quencher (BHQ1)


<220>
<221> misc_feature
<223> 5′-6-carboxyfluorescein is attached to the 5′ end and BHQ1
      quencher is attached to the 3′ end

<400> 13
tgggggcac agga                                                          14

## Description

### FIELD OF THE INVENTION

The present invention relates to the field of medicine, in particular of oncology. It provides genetic markers which can be used for the diagnosis, the prognosis or for the detection of predisposition to pancreatic cancers.

### BACKGROUND OF THE INVENTION

With more than 277,000 new cases per year in the world, pancreatic cancers (PC) represents some 10% of all digestive cancers among which 80-90 % are adenocarcinoma (PDAC). Among those newly diagnosed patients some 266,000 will die with an equal repartition between genders. Further prediction stipulates that this cancer will become the second cause of mortality by cancer by the end of the decade. The prognosis in part that of PDAC is dramatic with a 5-year survival rate of less than 4% in the Europe and in USA (Cartwright et al., 2008).

PDAC is still often diagnosed very late and the very poor prognosis of such cancer is mainly due to its lack of response to currently available therapies (Lockhart et al., 2005; Pliarchopoulou and Pectasides, 2009). Its curative resection rate is very low (15% of patients) due to unspecific symptoms, the lack of early specific biological markers, delayed diagnosis and metastasis formation and thrombotic events. Patients diagnosed with advanced or metastatic disease are not elected for surgery (85% of patients) and show median survival rates ranging from 6 to 9 months (Jarufe et al., 2005; Müller-Nordhorn et al., 2006). For those patients with inoperable cancer, the main treatment remains a standard chemotherapy (gemcitabine, and recently the multi-drug combination FOLFIRINOX comprising folinic acid, fluorouracil, irinotecan and oxaliplatin). However, to date, targeted therapies have shown limited clinical efficiency at best and PDAC clearly remains one of the most resistant neoplasia.

For pancreatic cancer, the size of the tumor and the involvement of lymph nodes are among the best prognostic factors; biological characteristics of the tumor and therapeutic strategies influence prognosis to a lesser extent. Early diagnosis is thus crucial to greatly increase the chance of survival from PDAC.

Recently, several genome-wide association studies identifying pancreatic cancer susceptibility loci were published and genetic variations in the loci 13q22.1, 1q32.1 and 5p15.33 (Petersen et al., 2010), 6p25.3, 12p11.21 and 7q36.2 (Low et al. 2010), 21q21.3, 5p13.1, 21q22.3, 22q13.32 and 10q26.11 (Wu et al. 2012) and in the locus ABO (9q34) (Amundadottir et al 2009,) were found to be associated to pancreatic cancers.

However, efforts are still pressing to identify valid biomarkers predictive of pancreatic cancer that would be useful for the identification of at-risk populations who will benefit from a personalized follow-up.

### SUMMARY OF THE INVENTION

The invention aims to provide valuable genetic markers to identify subjects having an increased risk of developing pancreatic cancers. Once these subjects have been identified, a follow-up protocol can be proposed in order to early diagnose pancreatic cancer, if occurred.

Accordingly, in a first aspect, the present invention relates to a method for determining a predisposition to, prognosing or diagnosing pancreatic cancer in a subject, comprising detecting the presence or absence of a genetic alteration in exon 11 of the BSDL gene, in a biological sample of said subject.

The genetic alteration in exon 11 may be a substitution, an insertion or a deletion of one or several bases. Preferably, the alteration is a single nucleotide polymorphism (SNP).

Preferably, the alteration is the SNP rs488087, or a polymorphic site in linkage disequilibrium with rs488087.

More preferably, the alteration is the SNP rs488087 and the identification of a T at polymorphism site rs488087, indicates an increased risk of developing pancreatic cancer.

The biological sample may be a tissue, urine, saliva, serum, plasma or whole blood sample.

The alteration may be detected by any method known by the skilled person, for example by sequencing, selective hybridization and/or selective amplification, enzyme-based methods or methods relying on differences in the conformation, weight or size of the molecules.

The pancreatic cancer is an exocrine or endocrine pancreatic cancer, preferably an exocrine pancreatic cancer.

More preferably, the pancreatic cancer is a pancreatic adenocarcinoma.

In a particular embodiment, the subject or patient has a personal history of diabetes, chronic pancreatitis or preneoplastic pancreatic lesions, a family history of pancreatic cancer or pancreatitis, or a particular hereditary condition such as BRCA1/2 mutations, Peutz-Jeghers syndrome, familial atypical mole and melanoma syndrome, von Hippel-Lindau syndrome, Lynch syndrome or Wermer Syndrome.

In a second aspect, the present invention relates to a kit comprising
(i) at least one nucleic acid probe capable of specifically binding or hybridizing to a genetic alteration in exon 11 of the BSDL gene, and/or
(ii) at least one set of primers suitable for use in amplification reaction, wherein the set of primers amplifies a genetic alteration in exon 11 of the BSDL gene,
   and optionally, a leaflet providing guidelines to use such a kit.

Preferably, the kit comprises
(i) at least one nucleic acid probe capable of specifically binding or hybridizing to the polymorphism site rs488087, and/or
(ii) at least one set of primers suitable for use in amplification reaction, wherein the set of primers amplifies the polymorphism site rs488087.

More preferably, the kit comprises a nucleic acid probe comprising, or consisting of, the sequence 5- TGGGGGGCACGGGA-3' (SEQ ID NO: 8), preferably attached to a fluorophore-quencher pair, and/or a nucleic acid probe comprising, or consisting of, the sequence 5'-TGGGGGGCACAGGA-3' (SEQ ID NO: 9), preferably attached to a fluorophore-quencher pair.

The kit may further comprise one or several reagents for detecting the hybridization of said at least one nucleic acid probe and/or for amplifying and/or detecting said genetic alteration or polymorphism site.

The present invention also relates to the use of the kit of the invention for determining a predisposition to, prognosing or diagnosing pancreatic cancer in a subject.

### BRIEF DESCRIPTION OF THE DRAWINGS

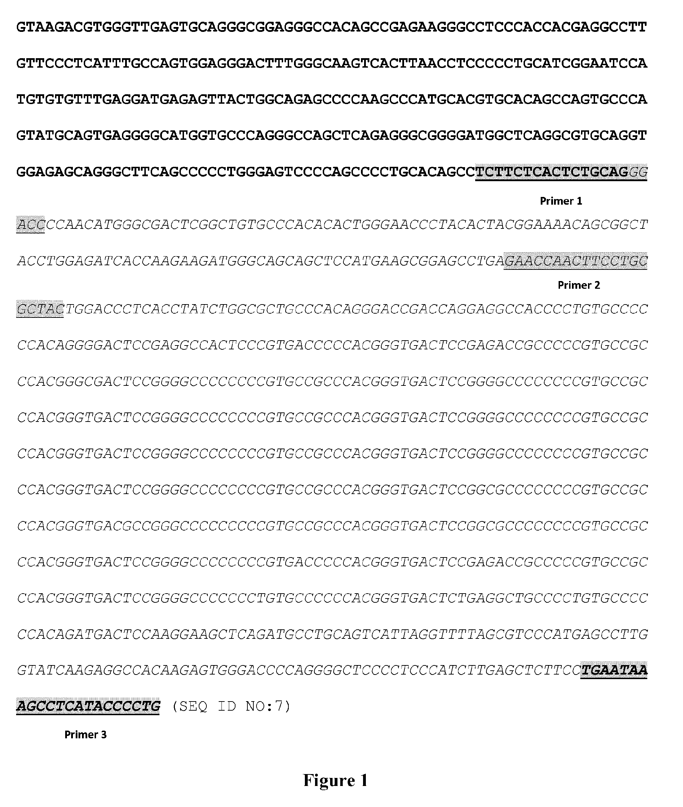

**Figure 1****:** Oligonucleotide positions in the BSDL sequence from intron 10 to the 3'end of exon 11. The intron 10 is indicated with bold letters. Exon 11 sequence starts with the last five nucleotides of primer 1 and ends with the bold italic letter sequence.

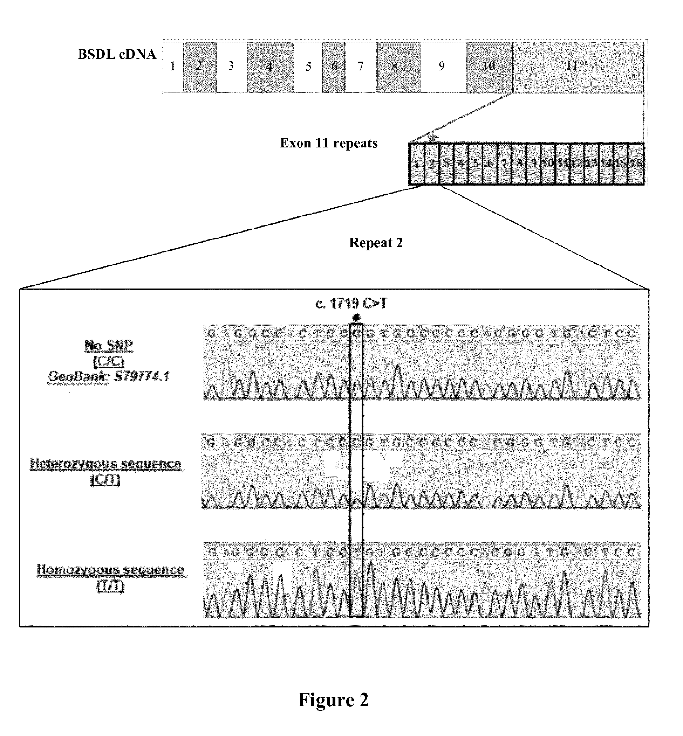

**Figure 2****:** Location of the SNP rs488087 within the cDNA sequence of BSDL. Top of the figure shows the 11 exons of the gene with the 16 repeated sequences. Chromatograms (lower panel) showing the position of the sample in a wild rs488087 sample (C/C), a heterozygous sample (C/T) and a homozygous (T/T) sample are shown.

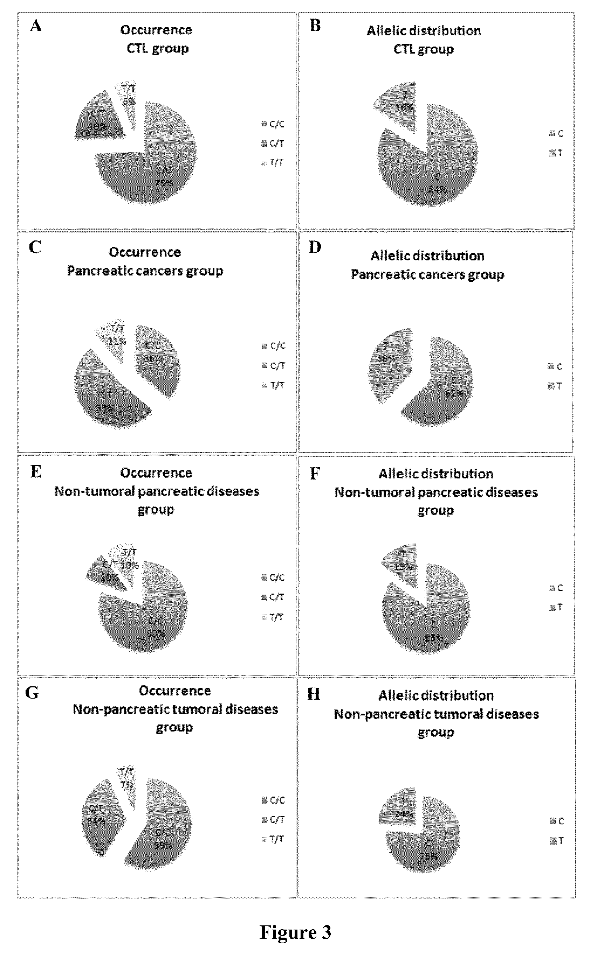

**Figure 3****:** Occurrence and allelic distribution of the Single Nucleotide Polymorphism (SNP) *rs488087* in the different groups.

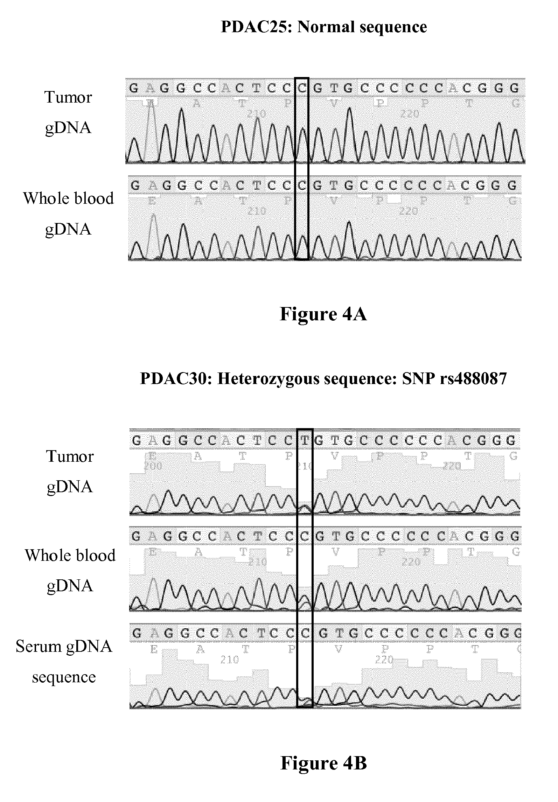

**Figure 4****:** Analysis of the chromatograms obtained after the sequencing of the amplicons by Sanger technique on several samples from the same patient. Fig. 4A: PDAC25: concordance between genomic DNA (gDNA) extracted from the tumor and the gDNA extracted from whole blood. Fig. 4B: PDAC30: concordance between gDNA extracted from the tumor, from the whole blood and from the serum.

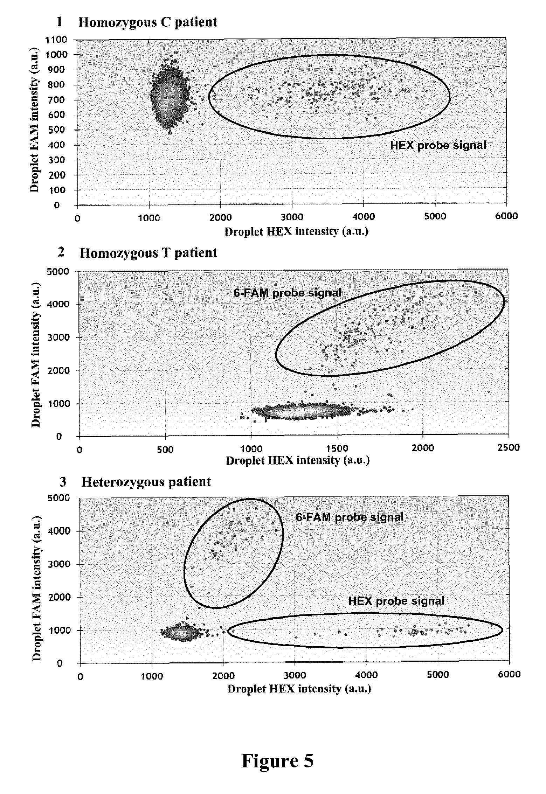

**Figure 5****:** Droplet Digital PCR (ddPCR) results analysis. HEX labeled probe recognize C target SNP and is visualized on X axis and 6-FAM labeled probe recognize T target SNP and is visualized on Y Axis. This graph shows results for three different patients (1) Homozygous C patient, (2) Homozygous T patients and (3) Heterozygous C/T patient.

### DETAILED DESCRIPTION OF THE INVENTION

The bile salt dependent lipase or BSDL is a glycoprotein mainly expressed in the acinar tissue of the pancreas (Lombardo 2001) and in lactating mammary glands (Blackberg et al. 1987). The pancreatic BSDL enzyme (E.C.3.1.1.13), also known as bile salt-stimulated or carboxyl-ester lipase (CEL), is secreted into the digestive tract where it is activated by the presence of primary bile salts, playing a role in cholesterol and lipid-soluble vitamin hydrolysis and absorption (Lombardo and Guy, 1980). A fraction of the enzyme is also present in plasma and an interaction with plasma cholesterol and oxidized lipoproteins has been suggested (Bengtsson-Ellmark et al. 2004). Furthermore, BSDL may be involved in angiogenesis (Augé et al. 2005), in vascular (Rebaï et al. 2005) and thrombotic events (Panicot-Dubois et al. 2007).

Human BSDL gene is about 10 kb, with a variable number of tandem repeats (VNTR) in the coding region of exon 11 (from 3 to 21 repeats). These tandem repeats are formed of 33-base pair (bp) nearly identical segments (Madeyski et al. 1998). Sixteen repeats are present on the most common allele of caucasian population (Raeder et al. 2006).

Previous studies have shown that O-glycosylation of repeated sequences (Wang et al. 1995) is not required for functional properties of the enzyme such as catalytic activity and activation by bile salts (Downs et al. 1994). However, the VNTR may be necessary for proper folding, secretion and stability (Bruneau et al. 1997). These sequences are also the target of the 16D10 and J28 monoclonal antibodies which are specific of pancreatic adenocarcinoma (Crescence et al. 2012) and of the oncofetal isoform of the enzyme referred to as the feto-acinar pancreatic protein or FAPP (Mas et al. 1993).

An association between the total number of repeats and serum cholesterol profile has been reported (Bengtsson-Ellmark et al. 2004). In addition, exocrine dysfunction often seen in diabetic patients, may be linked to common single-base insertions in the BSDL VNTR (Vesterhus et al. 2008). Two different single-base deletions, located in repeat 1 and repeat 4, were detected in two families with dominantly inherited diabetes and exocrine dysfunction. Both deletions lead to a frame shift and a premature stop codon, creating a new C-terminal end of the translated BSDL protein and to a misfolded protein responsible for the diabetic pathology (Johansson et al. 2011). Although pancreatic lipomatosis is associated with the disease (Raeder et al. 2007), the exact pathogenic effect of the mutated BSDL gene is still unknown. A study performed in a cohort of German patients showed the absence of relation between the numbers of VNTR and alcoholic or idiopatic chronic pancreatitis (Ragvin et al. 2013).

Furthermore, Reuss et al. investigated the pattern of BSDL (or CEL) gene expression in human adenocarcinomas and normal tissues of the pancreas and found that the lipase is not significantly transcribed in pancreatic adenocarcinomas. They thus concluded that BSDL gene is not an apt genetic marker for diagnostic or therapeutic approaches (Reuss et al., 2006).

The inventors herein demonstrated that a Single Polymorphism Nucleotide referred to as rs488087 (NCBI SNP database) and located in the sequence of the second VNTR of BSDL can be detected in blood, serum and tumor tissue of patients suffering with a PC. The occurrence of this SNP is highly significant when compared with the control cohort (63.9% vs 25.5 %, p = 0.0005). This SNP is detected at the same level in control than in non-tumoral pathologies of the pancreas (25.5% vs 20 %, Fisher's exact test, p = 1.00, *i.e.* not significant) or in non pancreatic tumoral diseases (25.5 % vs 40.9 %, Chi² test, p = 0.1189, *i.e.* not significant). These results demonstrate that the rs488087 germinal SNP is a new biomarker predictive of pancreatic cancer that can be used to identify populations having higher risk of developing pancreatic cancer than the general population and who should benefit from a follow-up in order to detect early signs of pancreatic cancer.

### Definitions

The term "cancer" or "tumor", as used herein, refers to the presence of cells possessing characteristics typical of cancer-causing cells, such as uncontrolled proliferation, immortality, metastatic potential, rapid growth and proliferation rate, and certain characteristic morphological features. This term refers to any type of malignancy (primary or metastases).

The term "pancreatic cancer" as used herein refers to endocrine and exocrine pancreatic cancers. Exocrine pancreatic cancers include, but are not limited to, pancreatic adenocarcinomas, adenosquamous carcinomas, squamous cell carcinomas, signet ring cell carcinomas, undifferentiated carcinomas, undifferentiated carcinomas with giant cells and solid pseudopapillary neoplasms of the pancreas, malignant endocrine carcinoma, mucinous cystadenoma and ampullary cancer (carcinoma of the ampulla of Vater). Endocrine pancreatic cancers include, but are not limited to, malignant insulinomas (from cells that make insulin), glucagonomas (from cells that make glucagon), gastrinomas (from cells that make gastrin), somatostatinomas (from cells that make somatostatin), VIPomas (from cells that make vasoactive intestinal peptide, i.e. VIP), and PPomas (from cells that make pancreatic polypeptide). Preferably, the pancreatic cancer is an exocrine pancreatic cancer, more preferably a pancreatic adenocarcinoma (PDAC).

As used herein the term "diagnosing" refers to determining presence or absence of pathology in a subject. More particularly, as used herein, this term refers to determining presence or absence of a pancreatic cancer, such as pancreatic adenocarcinoma, in a subject.

As used herein, the term "predisposition" or "genetic predisposition" refers to an increased susceptibility to, or a risk of developing, a particular disease, e.g. pancreatic cancer, due to the presence of one or several genetic alterations relative to a reference sequence. More particularly, this term refers to an increased susceptibility to pancreatic cancer due to the presence of a genetic alteration in exon 11 of the BSDL gene.

As used herein, the term "prognosing" refers to an assessment of the future outcome of a disease. In particular, this term means predicting whether a subject will suffer from a pancreatic cancer in the future.

The term "BSDL gene" as used herein, refers to a polynucleotide encoding a BSDL polypeptide (Gene ID: 1056). The bile salt dependent lipase or BSDL may also be named Bile salt-activated lipase (BAL), Bile salt-stimulated lipase (BSSL), Bucelipase, Carboxyl ester lipase (CEL or CELL), Cholesterol esterase (CEase), Pancreatic lysophospholipase, Sterol esterase, FAP, FAPP, LIPA or MODY8. An exemplary sequence for the precursor of this enzyme is Genbank Accession Number: NP_001798.2 (SEQ ID NO: 2). This precursor of 753 amino acids comprises a signal peptide of 20 amino acids. An exemplary sequence for the polynucleotide (mRNA) encoding this precursor is Genbank Accession Number: NM_001807.4 (SEQ ID NO:1). This polynucleotide comprises 11 exons. Exon 11 encodes a variable number of tandem repeats (VNTR), i.e. repeated C-terminal peptide sequences of 11 amino acids typically comprising a nearly identical part with 7 amino acids having the sequence Ala Pro Pro Val Pro Pro Thr and a glycosylation site. Said generally invariant part is flanked on either side by a glycine often substituted by a glutamic acid and contains the amino acids Asp and Ser on the N-terminal side. In the sequence set forth in SEQ ID NO: 1, exon 11 is from position 1510 to position 2386. However, as the gene contains a VNTR polymorphism (from 3 to 21 repeats), exon 11 of the BSDL gene from the subject may be shorter or longer.

The amino acids are herein represented by their one-letter or three-letter code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp) and Y: tyrosine (Tyr).

Herein, the terms "peptide", "oligopeptide", "polypeptide" and "protein" are employed interchangeably and refer to a chain of amino acids linked by peptide bonds, regardless of the number of amino acids forming said chain. The terms "nucleic acid", "oligonucleotide" and "polynucleotide" are employed interchangeably and refer to a chain of nucleotides regardless of the number of nucleotides forming said chain.

As used herein, the term "subject" or "patient" refers to a human, including adult, child and human at the prenatal stage.

The term "biological sample", as used herein, refers to any sample derived from a subject and containing nucleic acids. Examples of such samples include fluids such as whole blood, plasma, serum, saliva, urine and seminal fluid samples as well as biopsies, organs, tissues or cell samples. Most preferred samples are saliva, urine, whole blood, plasma, serum or tissue. The sample may be collected according to conventional techniques and used directly in the method of the invention or stored prior to its use. The sample may be treated prior to its use.

By "genetic alteration" is meant any alteration in a nucleic acid sequence relative to a reference. Genetic alterations include, but are not limited to, substitutions, insertions, deletions and frameshift mutations, and combinations thereof. Preferably, the genetic alterations is a substitution, an insertion or a deletion of one or several bases, e.g. of 1 to 50 bases. More preferably, the alteration is a single nucleotide polymorphism (SNP). The SNP may be a non-synonymous SNP (i.e. that alters the amino acid sequence of the encoded protein) or a synonymous SNP (i.e. which do not alter amino acid sequences).

The term "SNP" means "single nucleotide polymorphism". SNP can be referred by the location of the amino acid residue which is concerned or modified due to the polymorphism (e.g. Pro573Pro) or by the reference SNP number (e.g. rs488087) of the NCBI SNP database (http://www.ncbi.nlm.nih.gov/SNP).

As used herein, the term "reference sequence" is a defined sequence used as basis for sequence comparison. In particular, the reference sequence for detecting genetic alteration in exon 11 of the BSDL gene may be the sequence of the mRNA transcript of the BSDL gene, i.e. GenBank Accession number: NM_001807.4 (SEQ ID NO: 1) and in particular the region from position 1510 to position 2386 of this sequence (i.e. exon 11). In some embodiments, the reference sequence may also be the amino acid sequence of the BSDL protein and in particular the sequence set forth in SEQ ID NO: 2.

The methods of the invention as disclosed below may be *in vivo, ex vivo* or *in vitro* methods, preferably *in vitro* methods.

In a first aspect, the present invention relates to a method for determining a predisposition to, prognosing or diagnosing pancreatic cancer in a subject, comprising detecting the presence or absence of a genetic alteration in exon 11 of the BSDL gene, in a biological sample of said subject.

The method may further comprise providing a biological sample of the subject, preferably a saliva, urine, whole blood, serum or plasma sample, more preferably a whole blood, serum or plasma sample.

The genetic alteration in exon 11 may be a substitution, an insertion or a deletion of one or several bases, preferably located in the second repeat of the VNTR region.

In a particular embodiment, the genetic alteration in exon 11 is a SNP. Thus, in this embodiment, the method of the invention comprises genotyping the BSDL gene, or a part thereof comprising exon 11, to determine the presence of a SNP in exon 11.

In a preferred embodiment, the method comprises detecting the presence or absence of the SNP rs488087 (SEQ ID NO: 3), and/or a polymorphic site in linkage disequilibrium with rs488087.

The SNP rs488087 is located at position 1735 on NM_001807.4 reference (SEQ ID NO: 1) or at position 1719 starting from the ATG of the first methionin (at position 17) on NM 001807.4 reference which can be either a C or a T. This SNP is a synonymous polymorphism, i.e. the residue P573 of the SEQ ID NO: 2 is not altered.

In this embodiment, the presence of the SNP rs488087, i.e. the identification of a T at this polymorphism site, is deleterious for the subject and indicates an increased risk of developing pancreatic cancer or the presence of pancreatic cancer.

Linkage disequilibrium (LD) is defined as the non-random association of alleles at different loci across the genome. Alleles at two or more loci are in LD if their combination occurs more or less frequently than expected by chance in the population. When there is a causal locus in a DNA region, due to LD, one or more SNPs nearby are likely associated with the trait too. Therefore, any SNPs in strong LD (yielding a r²>0.6) with SNP rs488087 will be associated with this trait. Identification of additional SNPs in linkage disequilibrium with a given SNP involves: (a) amplifying a fragment from the genomic region comprising or surrounding a first SNP from a plurality of individuals; (b) identifying of second SNPs in the genomic region harboring or surrounding said first SNP; (c) conducting a linkage disequilibrium analysis between said first SNP and second SNPs; and (d) selecting said second SNPs as being in linkage disequilibrium with said first marker. Subcombinations comprising steps (b) and (c) are also contemplated.

Methods to identify SNPs and to conduct linkage disequilibrium analysis can be carried out by the skilled person without undue experimentation by using well-known methods.

It is well known that many SNPs have alleles that show strong LD with other nearby SNP alleles and in regions of the genome with strong LD, a selection of evenly spaced SNPs, or those chosen on the basis of their LD with other SNPs (proxy SNPs or Tag SNPs), can capture most of the genetic information of SNPs, which are not genotyped with only slight loss of statistical power. In association studies, this region of LD are adequately covered using few SNPs (Tag SNPs) and a statistical association between a SNP and the phenotype under study means that the SNP is a causal variant or is in LD with a causal variant. The two metrics most commonly used to measure LD are D' and r² and can be written in terms of each other and allele frequencies. It is a general consensus that a proxy (or Tag SNP) is defined as a SNP in LD (r² ≥ 0.8) with one or more other SNPs. The genotype of the proxy SNP could predict the genotype of the other SNP via LD and inversely. In particular, any SNP in LD with one of the SNPs used herein may be replaced by one or more proxy SNPs defined according to their LD as r² ≥ 0.8.

The method of the invention may comprise detecting the presence or absence of the SNP rs488087 and/or the presence or absence of one or several other SNPs in linkage disequilibrium with rs488087.

The genetic alteration in exon 11 of the BSDL gene may be determined at the level of the BSDL DNA, RNA or polypeptide, preferably at the level of DNA or RNA, by any method known by the skilled person.

These methods include, but are not limited to, direct sequencing-based methods, hybridization-based methods, primer extension-based methods, ligation-based methods, methods based on the conformation of a molecule containing the polymorphism, or invasive cleavage-based methods.

In a particular embodiment, the genetic alteration is detected by direct sequencing using well-known techniques, preferably using next-generation sequencing technologies. The sequencing may be performed on the complete sequence of the BSDL gene, or more preferably on the genomic region containing exon 11 of the BSDL.

In another embodiment, the genetic alteration is detected by selective hybridization. Hybridization based genotyping methods include, but are not limited to, southern hybridization, genotyping on a microarray, in particular on a SNP microarray (amplification products are analysed by hybridization to target sequences immobilized on a solid support), methods using molecular beacons designed to only hybridize to a specific allele, and dynamic allele-specific hybridization (DASH) assay.

In another embodiment, the genetic alteration is detected by selective amplification. Amplification may be performed according to various techniques known in the art, such as by polymerase chain reaction (PCR), Touch-down PCR (TD-PCR), droplet-digital PCR (ddPCR), quantitative PCR (q-PCR), ligase chain reaction (LCR), strand displacement amplification (SDA) and nucleic acid sequence based amplification (NASBA). These techniques can be performed using commercially available reagents and protocols. Preferred techniques use allele-specific PCR, tetra-primer ARMS-PCR (Shu et al., 2001).

The genetic alteration may also be determined by enzyme based methods including, but not limited to, restriction fragment length polymorphism (RFLP) analysis, invader assay relaying on the activity of flap endonuclease that cleaves at specific nucleic acid structures, enzymatic digestion, TaqMan assay or 5'-nuclease probe assay relying on the 5'-nuclease activity of Taq DNA polymerase (e.g. implemented using droplet-digital PCR (ddPCR) or quantitative PCR (q-PCR)); oligonucleotide ligation assay, ligation rolling circle amplification or L-RCA (Xiaoquan et al., 2001), or primer extension based methods relying on the hybridization of a probe to the bases immediately adjacent to the specific polymorphic allele.

The genetic alteration may also be determined using methods relying on the differences in conformation, weight, or size of molecules such as PCR-SSCP, MALDI-TOF mass spectrometry (Griffin and Smith, 2000), denaturing-HPLC and temperature gradient gel electrophoresis (TGCE).

The various methods may be carried out in various reaction formats including homogeneous reactions and reactions on solid supports. Various detection methodologies may be employed in detecting genetic alterations including, but not limited to, radioactive detection, luminescence detection, fluorescence detection, time-resolved fluorescence detection, fluorescence resonance energy transfer, fluorescence polarization, gel electrophoresis, and mass spectrometry.

Preferably, the genetic alteration in exon 11 of the BSDL gene is determined using at least one nucleic acid probe hybridizing to a specific allele.

In a particular embodiment, the genetic alteration is the SNP rs488087 and is detected using a nucleic acid probe specifically hybridizing to the T allele and/or a nucleic acid probe specifically hybridizing to the C allele, at the polymorphic site rs488087. Preferably, the allele at the polymorphic site rs488087 is determined with a 5'-nuclease probe assay, using a nucleic acid probe specifically hybridizing to the T allele and/or a nucleic acid probe specifically hybridizing to the C allele, in particular using any probe described below as part of the kit of the invention.

It is known that the health history of a subject can affect the risk of pancreatic cancer. In particular, familial factors are known to be involved in susceptibility to pancreatic cancer. Indeed, whereas the lifetime risk of pancreatic cancer is of approximately 1 percent in the general population, it reaches 4.7 percent for first-degree relatives of pancreatic cancer cases and increases with each family member who is affected. Furthermore, in some cases, pancreatic cancer can be inherited as part of a multi-cancer syndrome, such as that associated with BRCA1/2 mutations, Peutz-Jeghers syndrome, familial atypical mole and melanoma syndrome, von Hippel-Lindau syndrome, Lynch syndrome or Wermer Syndrome.

Thus, in an embodiment, the subject has a personal history of diabetes, chronic pancreatitis, preneoplastic pancreatic lesions or relapses of pancreatic cancers, a family history of pancreatic cancer or pancreatitis, or a particular hereditary condition.

However, the vast majority of pancreatic cancers are sporadic. Thus, in another embodiment, the subject does not have any personal or family history of diabetes or other pancreatic pathologies.

When the subject is diagnosed with pancreatic cancer or identified as having a risk of developing a pancreatic cancer according to the method of the invention, appropriate treatment may be prescribed.

In a particular embodiment, the method of the invention thus comprises an additional step of treating the subject diagnosed with pancreatic cancer or identified as having a risk of developing a pancreatic cancer.

As used herein, the term "treatment" or "therapy" includes curative and/or prophylactic treatment. More particularly, curative treatment refers to any of the alleviation, amelioration and/or elimination, reduction and/or stabilization (e.g., failure to progress to more advanced stages) of a symptom, as well as delay in progression of a symptom of a pancreatic cancer. Such curative treatment may include surgery, radiotherapy and/or chemotherapy.

The term "radiotherapy" is commonly used in the art to refer to multiple types of radiation therapy including internal and external radiation therapy, radio immunotherapy, and the use of various types of radiation including X-rays, gamma rays, alpha particles, beta particles, photons, electrons, neutrons, radioisotopes, and other forms of ionizing radiation. Preferably, the radiotherapy involves the use of X-rays or gamma-rays.

As used herein, the term "chemotherapy" refers to a cancer therapeutic treatment using chemical or biochemical substances, in particular using one or several antineoplastic agents. In particular, the chemotherapy may involve the use of at least one antineoplastic agent selected from the group consisting of gemcitabine, Erlotinib or Sunitinib preferably in combination with gemcitabine, Everolimus, Mitomycin C, Abraxane (paclitaxel protein-bound particles for injectable suspension) preferably in combination with gemcitabine, OFF (combination of oxaliplatin, fluorouracil and folinic acid), Folfirinox (combination of oxaliplatin, irinotecan, fluorouracil and folinic acid), and any combination thereof.

Prophylactic treatment or "prevention" refers to any of: halting the onset, reducing the risk of development, reducing the incidence, delaying the onset, reducing the development, as well as increasing the time to onset of symptoms of a pancreatic cancer. In the context of the present invention, the term "preventing" more particularly applies to a subject who is at risk of developing a pancreatic cancer and includes for example a periodic follow-up of the subject to early detect the occurrence of pancreatic cancer and thus to improve prognosis.

In particular, the subject who is at risk of developing a pancreatic cancer may be submitted to follow-up tests each year, each 2, 3, 4, or 5 years. Follow-up tests usually include tests and procedures that make pictures of the pancreas and the area around it. In particular, these tests may comprise a physical exam to check general signs of health, biochemical analysis, such as blood analysis and/or tumor marker test, magnetic resonance imaging, CT scan, PET scan, abdominal ultrasound, endoscopic ultrasound, endoscopic retrograde cholangiopancreatography, percutaneous transhepatic cholangiography, laparoscopy or biopsy.

In another aspect, the present invention also relates to a kit comprising
(i) at least one nucleic acid probe capable of specifically binding or hydridizing to a genetic alteration in exon 11 of the BSDL gene, and/or
(ii) at least one set of primers suitable for use in amplification reaction, wherein the set of primers amplifies a genetic alteration in exon 11 of the BSDL gene,
and optionally, a leaflet providing guidelines to use such a kit.

The set of primers may allow amplification of the BSDL gene in its entirety, of the exon 11, or only a fragment of exon 11 comprising the genetic alteration.

In a preferred embodiment, the kit comprises
(i) at least one nucleic acid probe capable of specifically binding or hybridizing to the polymorphism site rs488087, and/or
(ii) at least one set of primers suitable for use in amplification reaction, wherein the set of primers amplifies the polymorphism site rs488087.

In a particular embodiment, the set of primer may comprise one or several primers selected from the group consisting of SEQ ID NO: 4 to 6, 10 and 11.

In a preferred embodiment, the kit comprises at least one nucleic acid probe capable of specifically binding or hybridizing to the polymorphism site rs488087. In particular, the kit may comprise a nucleic acid probe comprising, or consisting of, the sequence 5-TGGGGGGCACGGGA-3' (SEQ ID NO: 8), and/or a nucleic acid probe comprising, or consisting of, the sequence 5'-TGGGGGGCACAGGA-3' (SEQ ID NO: 9). Preferably, the kit comprises at least these two probes.

In some embodiments, the nucleic acid probes may be attached to at least one reporter molecule generating a detectable signal. The detectable signal may be for example a fluorescent, luminescent or colored signal.

In preferred embodiments, the nucleic acid probes are attached to a fluorophore-quencher pair. These probes are particularly useful in 5'-nuclease probe assays. 5'-nuclease probes are single-stranded hybridization probes labeled with a donor-acceptor fluorophore pair that interact via FRET. The probe is designed to hybridize to its target DNA strand at the same time as the PCR primer. When Taq DNA polymerase extends the primer, it encounters the probe, and as a result of its 5'-nuclease activity, it cleaves the probe. Cleavage of the probe results in the separation of the donor fluorophore and quencher molecule, and leads to an increase in the intensity of the fluorescence signal.

A wide variety of fluorophore-quencher pairs are detailed in the literature (see .e.g. Marras et al. Methods Mol Biol. 2006;335:3-16) and in supplier's catalogues.

Examples of fluorophores include, but are not limited to, fluorescein derivatives such as 5-carboxyfluorescein (5-FAM), 6-carboxyfluorescein (6-FAM), tetrachlorofluorescein (TET) and hexachlorofluorescein (HEX), cyanine dyes such as Cy2, Cy3 and Cy5, Tetramethylrhodamine (TMR), Oregon Green dyes and Texas Red.

Examples of quenchers include, but are not limited to, DABCYL (4-(4-(dimethylamino) phenylazo) benzoic acid), QSY™ quenchers (Invitrogen, Carlsbad, CA), ECLIPSE™ quenchers (Epoch Biosciences, Bothell, WA) and BHQ™ (BHQ-I, 2, and 3 (Biosearch Technologies, Inc. Novato CA)) and DDQ-1 or -2.

In particular embodiments, the fluorophore-quencher pair is selected from HEX-BHQ1 and 6-FAM-BHQ1. Preferably, distinct fluorophore-quencher pairs are attached to each nucleic acid probe type, i.e. probes specific to the C allele and probes specific to the T allele at the polymorphic site rs488087, in order to distinguish their signals.

In particular, the nucleic acid probe specific to the C allele may comprise, or consist of, the sequence set forth in SEQ ID NO: 8, and may further comprise hexachlorofluorescein attached to its 5' end and BHQ1 quencher attached to its 3' end (SEQ ID NO : 12).

The nucleic acid probe specific to the T-allele may comprise, or consist of, the sequence set forth in SEQ ID NO: 9 and may further comprise 6-carboxyfluorescein attached to its 5' end and BHQ1 quencher attached to its 3' end (SEQ ID NO : 13).

The kit may also comprise at least one molecular beacon or a microarray that can be used to detect the SNP rs488087.

The kit may further comprise one or several reagents for detecting the hybridization of said at least one nucleic acid probe and/or for amplifying and/or detecting said genetic alteration or polymorphism site. In a particular embodiment, the kit further comprise reagents needed to perform droplet digital PCR.

The present invention also relates to the use of this kit for determining a predisposition to, prognosing or diagnosing pancreatic cancer in a subject, according to the method of the invention.

All embodiments disclosed above for the method of the invention are also contemplated in this aspect.

Further aspects and advantages of the present invention will be described in the following examples, which should be regarded as illustrative and not limiting.

### EXAMPLES

### Example 1

### MATERIALS AND METHODS

### Pancreatic cancer (PC) samples.

The tissue samples were obtained after pancreatic resection (duodeno-pancreatectomy) in patients with pancreatic cancers either with adenocarcinoma (PDAC; 31 patients), or non-PDAC pancreatic cancers such as ampulloma, malignant endocrine carcinoma, mucinous cystadenoma (n=5). All these patients constitute the pancreatic cancer group (n = 36).

For patients with pancreatic adenocarcinoma, serum or plasma samples were collected systematically. For some patients, whole blood samples were also collected.

### Non-tumoral pancreatic disease (non-TPD) samples.

Non-tumoral pancreatic tissues with chronic calcifying pancreatitis or intraductal papillary mucinous tumor (CCP or IPMT; 10 patients) came from the CRO2 (Center for Research in Oncobiology and Oncopharmacology) biobank. All these patients constitute the non-tumoral pancreatic diseases group (n = 10).

### Non-pancreatic cancer (OC for Other Cancers) samples.

A cohort of patients suffering with non-pancreatic cancer tumoral pathologies such as glioblastoma, sarcoma and intestinal carcinoma, was used as non-pancreatic cancer group (n = 44).

All tissues samples were obtained in accordance with ethical guidelines and stored in the CRO2 biobank (agreement DC 2013-1857) or AP-HM ("Assistance Publique - Hôpitaux de Marseille) BioBank (agreement AC-2013-1786). They were kept in a solution of RNA later® (Life Technologies) immediately after surgery and then subjected to flash freezing in liquid nitrogen.

### Control group (CTL)

The control group was formed from 47 individuals presenting with no gastrointestinal or cancer pathologies. These blood samples of control patients were obtained in accordance with ethical guidelines.

**Table 1: Data about the control group**

| **Individuals** | **SNP rs 488087** | | **Individuals** | **SNP rs 488087** |
|---|---|---|---|---|
| **CTL 1** | **C/T** | | **CTL 25** | **T/T** |
| **CTL 2** | **C/C** | | **CTL 26** | **C/T** |
| **CTL 3** | **C/C** | | **CTL 27** | **C/C** |
| **CTL 4** | **C/T** | | **CTL 28** | **C/C** |
| **CTL 5** | **C/C** | | **CTL 29** | **C/T** |
| **CTL 6** | **C/C** | | **CTL 30** | **C/C** |
| **CTL 7** | **C/T** | | **CTL 31** | **C/C** |
| **CTL 8** | **C/C** | | **CTL 32** | **C/T** |
| **CTL 9** | **C/C** | | **CTL 33** | **C/T** |
| **CTL 10** | **C/C** | | **CTL 34** | **C/C** |
| **CTL 11** | **C/C** | | **CTL 35** | **C/C** |
| **CTL 12** | **C/C** | | **CTL 36** | **C/C** |
| **CTL 13** | **C/C** | | **CTL 37** | **C/C** |
| **CTL 14** | **C/T** | | **CTL 38** | **C/C** |
| **CTL 15** | **C/C** | | **CTL 39** | **T/T** |
| **CTL 16** | **C/C** | | **CTL 40** | **C/C** |
| **CTL 17** | **C/C** | | **CTL 41** | **C/C** |
| **CTL 18** | **C/C** | | **CTL 42** | **C/C** |
| **CTL 19** | **C/C** | | **CTL 43** | **C/C** |
| **CTL 20** | **C/T** | | **CTL 44** | **C/C** |
| **CTL 21** | **C/C** | | **CTL 45** | **C/C** |
| **CTL 22** | **C/C** | | **CTL 46** | **C/C** |
| **CTL 23** | **T/T** | | **CTL 47** | **C/C** |
| **CTL 24** | **C/C** | | | |

### Extraction of genomic DNA (gDNA) and total RNA from tissue and blood samples

The gDNA was extracted from whole blood, serum and frozen tissue using the "QIAamp mini kit" (Qiagen, Chatsworth, Calif., USA). The extraction is carried out using 600 µL of whole blood, serum or 25 mg of tissue as recommended by the supplier. The quantity and quality of extracted gDNA were determined by measuring the absorbance at 260 nm and 280 nm on LVisPlate Omega (BMG Labtech, Champigny s / Marne, France). gDNA samples were stored at -80°C until use.

The extraction of total RNA was performed using the "RNeasy® Plus Mini kit®" (Qiagen) according to the protocol recommended by the supplier. Total RNA was quantified by optical density at 260 nm on LVisPlate Omega. The quality and integrity of extracted RNA was verified by agarose gel migration. Total RNA samples were then stored at -80°C until use.

### Pre-Amplification of gDNA extracted from serum or from plasma.

gDNA samples of serum or plasma with concentrations lower than 10 ng, required a WGA (whole genome amplification). This pre-amplification was performed using the "RepliG mini kit" (Qiagen) according to the supplier's recommendations.

### Reverse transcription and amplifications by TD-PCR.

### Reverse transcription

Reverse transcription was performed from 1µg of total RNA in a reaction volume of 20µl, containing 0.4 mM Oligo(dT)₁₅ primer, 2 mM dNTP mix, 5 mM MgCl2, 20 U RNase inhibitor and 10 U AMV reverse transcriptase ("AMV Reverse Transcriptase" kit, Promega, France). The mixture was incubated 10 min at room temperature, followed by one hour at 42°C, 5 min at 99°C and finally 10 min in ice.

### Touch-Down Polymerase Chain Reaction (TD-PCR) amplification of cDNA or gDNA

Amplifications of sequences encoding BSDL were made either from cDNA, or genomic DNA from pancreatic tissue, whole blood or serum samples using specific primers (Eurogenetech). Primers used for amplification are given in Table 2 and their location in the BSDL sequence is presented in Figure 1 (SEQ ID NO: 7).

**Table 2: The primers used for amplification of sequences encoding the BSDL:**

| | cDNA | gDNA |
|---|---|---|
| Sense primers | 5'GAACCAACTTCCTGCGCTAC 3' (SEQ ID NO : 4) : primer 2 | 5'TCTTCTCACTCTGCAGGGA CC 3' (SEQ ID NO : 5) : primer 1 |
| Antisense primer | 5'CAGGGGTATGAGGCTTTATTCA 3' (SEQ ID NO : 6) : primer 3 | |

The TD-PCR was performed in a personal Mastercycler thermocycler (Eppendorf) in a reaction volume of 50 µl and comprises 100 ng gDNA or 2 µl of cDNA, 5 µl of 10X enzyme buffer, 10 µl of 5X GCmelt buffer (Ozyme, St Quentin en Yvelines, France), 200 nM of each primer, 0.4 mM of dNTP mix and 5 U Platinum Taq High fidelity DNA polymerase (Life Technologies, Saint Aubin, France). The program was applied as follows: 1 cycle of 5 min at 94°C; 9 cycles of 30 sec at 94°C, 30 sec at 64°C (-1°C per cycle), 1 min at 68°C; 30 cycles of 30 sec at 94°C, 30 sec at 55°C, 1 min at 68°C; 1 cycle of 12 min at 68°C. At the end of PCR, migration on agarose gel was performed to visualize the amplified fragment and check sizes.

### Amplicons purification

The amplicons obtained were then purified using the "DNA extraction" kit (Millipore, Molsheim, France) after electrophoretic migration on agarose gel according to the protocol recommended by the supplier.

### PCR products sequencing

Sequencing of the PCR products was carried out according to the Sanger method by Beckman Coulter Genomics (Meylan, France).

### Statistics

For statistical analysis of results, the exact Fischer test or Chi² test were performed using the Prism software.

### RESULTS

### Clinical data

### Pancreatic cancer (PC) group.

Tumor tissues came from 36 patients diagnosed with a pancreatic cancer between February 2007 and February 2014 (Table 3). Patients were aged between 50 and 87 years (mean = 66.7 years, SD =26.16) and the male / female ratio was 3/4. Definitive diagnosis of pancreatic cancer (adenocarcinoma) was given after immunohistochemical analysis. The average tumor size was 3.15 cm (SD = 3.3). WHO and TNM stages have been determined by a pathologist expert in the field. A patient had a localized tumor stage 1B, 10 patients had a localized tumor stage 2A, 18 patients had lymph node metastases (stage 2B), 5 patients are diagnosed at stage 4 (metastatic stage) and 2 patients NC. Survival time ranges were from 3 to 72 months. According to hospital records in 2014, 22 out of 36 patients with PC died and 9 are still alive. Information is lacking for remaining patients.

**Table 3: Clinical data of patients with pancreatic cancers (PC)**

| **Patients** | **Diseases** | Age (years) | Gender (M :0, F :1) | Tumor diameter (cm) | TNM (T : tumor, N : node, M : metastasis) | | | Grade | KRAS | **SNP rs488087** |
|---|---|---|---|---|---|---|---|---|---|---|
| | | | | | T | N | M | | | |
| **PC 1** | **PDAC** | 59 | 0 | 4.5 | 2 | 1 | 0 | 2B | - | **C/T** |
| **PC 2** | **PDAC** | 66 | 0 | 3.5 | 3 | 0 | 0 | 2A | - | **C/T** |
| **PC 3** | **PDAC** | 54 | 0 | 3 | 3 | 1 | 0 | 4 | G12D | **C/C** |
| **PC 4** | **PDAC** | 73 | 1 | 1.8 | 3 | 1 | 0 | 2B | - | **C/T** |
| **PC** 5 | **PDAC** | 62 | 0 | 2 | 3 | 1 | 0 | 2B | - | **C/C** |
| **PC 6** | **PDAC** | 57 | 1 | 4.3 | 4 | 1 | 1 | 4 | G12R | **T/T** |
| **PC 7** | **Mucinous cystadenoma** | 69 | 0 | NC | NC | | | NC | NC | **C/T** |
| **PC 8** | **PDAC** | 70 | 0 | 2.3 | 3 | 1 | 0 | 2B | G12V | **C/C** |
| **PC 9** | **PDAC** | 63 | 1 | 2.5 | 3 | 1 | 0 | 2B | - | **C/T** |
| **PC 10** | **PDAC** | 66 | 0 | 6 | 3 | 0 | 0 | 2A | - | **C/T** |
| **PC 11** | **PDAC** | 58 | 1 | 5.2 | 2 | 1 | 0 | 2B | - | **C/C** |
| **PC 12** | **PDAC** | 67 | 1 | 1.5 | 3 | 0 | 0 | 2A | G12D | **C/T** |
| **PC 13** | **PDAC** | 64 | 0 | 4.7 | 3 | 1 | 1 | 4 | G12D | **C/T** |
| **PC 14** | **PDAC** | 57 | 1 | 6 | 3 | 1 | 0 | 2B | - | **T/T** |
| **PC 15** | **PDAC** | 63 | 1 | 4.5 | 3 | 0 | 0 | 2B | G12D | **C/C** |
| **PC 16** | **PDAC** | 66 | 1 | 2.5 | 3 | 0 | 0 | 2A | - | **C/T** |
| **PC 17** | **PDAC** | 66 | 1 | 2.5 | 3 | 0 | 0 | 2A | G12R-D54Y | **C/T** |
| **PC 18** | **Malignant ampulloma** | 73 | 1 | 2,4 | 4 | 1 | 0 | NC | NC | **C/T** |
| **PC 19** | **PDAC** | 79 | 1 | 2 | 3 | 0 | 0 | 2A | - | **C/C** |
| **PC 20** | **PDAC** | 57 | 0 | 3 | 3 | 0 | 0 | 2A | G12V | **C/C** |
| **PC 21** | **Endocrine carcinoma** | 81 | 1 | 3 | 3 | 1 | 0 | NC | NC | **C/T** |
| **PC 22** | **PDAC** | 62 | 0 | 3 | 3 | 0 | 0 | 2A | - | **C/C** |
| **PC 23** | **Endocrine carcinoma** | 53 | 1 | 2 | 1 | 0 | 0 | 2A | - | **C/T** |
| **PC 24** | **PDAC** | 67 | 0 | 1.3 | 3 | 1 | 0 | 2B | - | **C/T** |
| **PC 25** | **PDAC** | 61 | 1 | 2.3 | 3 | 1 | 0 | 2B | - | **C/C** |
| **PC 26** | **Malignant ampulloma** | 60 | 1 | 2,5 | 4 | 1 | 0 | NC | NC | **C/C** |
| **PC 27** | **PDAC** | 72 | 0 | 3 | 3 | 1 | 0 | 2B | - | **T/T** |
| **PC 28** | **PDAC** | 69 | 1 | 3.5 | 3 | 0 | 0 | 2A | G12D | **C/T** |
| **PC 29** | **PDAC** | 57 | 1 | NC | 3 | 1 | 0 | 2B | NC | **C/C** |
| **PC 30** | **PDAC** | 73 | 1 | 2 | 2 | 0 | 0 | 1B | - | **T/T** |
| **PC 31** | **PDAC** | 50 | 1 | 3 | 2 | 1 | 0 | 2B | - | **C/T** |
| **PC 32** | **PDAC** | 87 | 1 | 3 | 3 | 1 | 0 | 2B | NC | **C/C** |
| **PC 33** | **PDAC** | 85 | 0 | 3.3 | 3 | 1 | 0 | 2B | NC | **C/T** |
| **PC 34** | **PDAC** | 79 | 1 | 3.5 | 3 | 1 | 0 | 2B | NC | **T/T** |
| **PC 35** | **PDAC** | 73 | 1 | 4.1 | 3 | 1 | 0 | 2B | NC | **C/C** |
| **PC 36** | **Endocrine carcinoma** | 83 | 0 | NC | NC | | | NC | NC | **C/T** |

| | | | | | | | | | | |
|---|---|---|---|---|---|---|---|---|---|---|
| NC: data not communicated. PC: Pancreatic Cancer, PDAC: pancreatic ductal adenocarcinoma, SNP: single nucleotide polymorphism. | | | | | | | | | | |

### Non-tumoral pancreatic diseases (non-TPD) group

The cohort of patients with non-tumoral pathology of the pancreas comprised 10 patients aged between 40 and 76 years (mean age = 64.5 years, SD = 25.4) (Table 4). Three patients had intraductal papillary mucinous tumor of the pancreas (IPMT), i.e. a cystic tumor of the pancreas with a putative pre-neoplastic statute. Six patients suffered from chronic calcifying pancreatitis (CCP), i.e. a benign disease of the pancreas. One patient presented retention cyst which is a complication of the CCP. According to the hospital records, all these patients were still alive by the end of 2013.

**Table 4: Clinical data of the cohort of patients with non-tumoral pancreatic diseases (non-TPD).**

| **Patients** | **Diseases** | Age (years) | Gender (M: 0, F:1) | **SNP rs488087** |
|---|---|---|---|---|
| **non-TPD 1** | **IMPT** | 69 | 0 | **C/C** |
| **non-TPD 2** | **IMPT** | 56 | NC | **C/C** |
| **non-TPD 3** | **CCP** | 61 | NC | **T/T** |
| **non-TPD 4** | **CCP** | 72 | 0 | **C/C** |
| **non-TPD 5** | **CCP** | 71 | 1 | **C/T** |
| **non-TPD 6** | **Retention cyst** | 76 | 1 | **C/C** |
| **non-TPD 7** | **CCP** | 40 | 1 | **C/C** |
| **non-TPD 8** | **CCP** | 73 | 0 | **C/C** |
| **non-TPD 9** | **CCP** | 53 | 1 | **C/C** |
| **non-TPD 10** | **IMPT** | 74 | 1 | **C/C** |

| | | | | |
|---|---|---|---|---|
| NC: data not communicated. IMPT, intra-papillary mucinous tumor of the pancreas; CCP chronic calcifying pancreatitis | | | | |

### Non-pancreatic tumoral disease (Other Cancers: OC) group.

A cohort of 44 patients with non-pancreatic malignancies was examined. In this cohort 40 patients were diagnosed with glioblastomas (G), 2 with sarcomas (S) one with an intestinal cancer with pancreatic metastases (I) and 1 with pancreato-biliary cancer [aged between 20.5 and 78.2 years (male / female = 1/3 ratio)] (Table 5).

**Table 5: Clinical data of the cohort of patients with non-pancreatic malignancies.**

| **Patients** | **Diseases** | Age (years) | Gender (M:0, F: 1) | Grade | P53 mutée (Amplified: 1, Normal :0) | EGFR (Amplified: 1 , Normal :0) | **SNP *rs488087*** |
|---|---|---|---|---|---|---|---|
| **OC 1** | **Glioblastoma** | 78.2 | 0 | 4 | 1 | 1 | **C/T** |
| **OC 2** | **Glioblastoma** | 20.5 | 0 | 4 | 1 | 0 | **C/T** |
| **OC 3** | **Pancreatobiliary cancer** | 64 | 0 | T3N1M0 | NC | NC | **C/C** |
| **OC 4** | **Glioblastoma** | 59.4 | 0 | 4 | 1 | 1 | **C/C** |
| **OC 5** | **Glioblastoma** | 52.6 | 0 | 4 | 0 | 0 | **C/C** |
| **OC 6** | **Glioblastoma** | 51.4 | 0 | 4 | 1 | 0 | **C/C** |
| **OC 7** | **Glioblastoma** | 63.1 | 0 | 4 | 0 | 0 | **T/T** |
| **OC 8** | **Glioblastoma** | 76.3 | 0 | 4 | 1 | 0 | **C/T** |
| **OC 9** | **Sarcoma GIST** | 42 | 1 | NC | NC | NC | **C/C** |
| **OC 10** | **Glioblastoma** | 48.7 | 0 | 4 | 0 | 1 | **C/C** |
| **OC 11** | **Glioblastoma** | 72.8 | 0 | 4 | 1 | 0 | **C/C** |
| **OC 12** | **Glioblastoma** | 56.1 | 0 | 4 | 1 | 1 | **T/T** |
| **OC 13** | **Glioblastoma** | 54.5 | 0 | 4 | 1 | 0 | **C/C** |
| **OC 14** | **Glioblastoma** | 39.8 | 0 | 4 | 1 | 0 | **C/C** |
| **OC 15** | **Glioblastoma** | 37.2 | 1 | 4 | 1 | 1 | **C/C** |
| **OC 16** | **Glioblastoma** | 75.4 | 0 | 4 | 1 | 0 | **C/C** |
| **OC 17** | **Glioblastoma** | 68.9 | 0 | 4 | 1 | 0 | **C/C** |
| **OC 18** | **Glioblastoma** | 46.6 | 0 | 4 | 1 | 1 | **C/C** |
| **OC 19** | **Glioblastoma** | 77 | 1 | 4 | 1 | 0 | **C/C** |
| **OC 20** | **Glioblastoma** | 49.8 | 0 | 4 | 0 | 1 | **C/C** |
| **OC 21** | **Glioblastoma** | 48.9 | 1 | 4 | 1 | 1 | **C/C** |
| **OC 22** | **Glioblastoma** | 59.5 | 1 | 4 | 0 | 0 | **C/C** |
| **OC 23** | **Glioblastoma** | 68.8 | 0 | 4 | 1 | 1 | **C/T** |
| **OC 24** | **Glioblastoma** | 53.3 | 0 | 4 | 0 | 1 | **C/C** |
| **OC 25** | **Glioblastoma** | 53.6 | 0 | 4 | 0 | 1 | **C/T** |
| **OC 26** | **Glioblastoma** | 59.1 | 1 | 4 | 1 | 0 | **C/C** |
| **OC 27** | **Glioblastoma** | 69.8 | 0 | 4 | 0 | 1 | **C/C** |
| **OC 28** | **Glioblastoma** | 73.1 | 0 | 4 | 1 | 0 | **C/T** |
| **OC 29** | **Glioblastoma** | 63,6 | 1 | 4 | 1 | 1 | **C/T** |
| **OC 30** | **Glioblastoma** | 70,9 | 1 | 4 | 0 | 1 | **C/T** |
| **OC 31** | **Glioblastoma** | 51,5 | 1 | 4 | 0 | 1 | **C/C** |
| **OC 32** | **Sarcoma GIST** | 34 | 0 | NC | NC | NC | **C/C** |
| **OC 33** | **Glioblastoma** | 69,1 | 0 | 4 | 1 | 0 | **C/C** |
| **OC 34** | **Glioblastoma** | 67,6 | 0 | 4 | 0 | 0 | **C/T** |
| **OC 35** | **Glioblastoma** | 54,6 | 1 | 4 | 0 | 0 | **C/T** |
| **OC 36** | **Glioblastoma** | 64,4 | 0 | 4 | 0 | 1 | **C/T** |
| **OC 37** | **Glioblastoma** | 75,4 | 0 | 4 | 1 | 0 | **C/T** |
| **OC 38** | **Glioblastoma** | 45,5 | 1 | 4 | 1 | 1 | **C/C** |
| **OC 39** | **Glioblastoma** | 59,2 | 0 | 4 | 1 | 1 | **C/T** |
| **OC 40** | **Glioblastoma** | 68,1 | 0 | 4 | 0 | 0 | **C/T** |
| **OC 41** | **Glioblastoma** | 61,3 | 0 | 4 | 1 | 0 | **C/T** |
| **OC 42** | **Glioblastoma** | 55,6 | 0 | 4 | 1 | 0 | **C/T** |
| **OC 43** | **Glioblastoma** | 51,9 | 0 | 4 | 1 | 1 | **C/C** |
| **OC 44** | **intestinal cancer** | 76 | 0 | T2 N0 M0 | NC | NC | **C/C** |

| | | | | | | | |
|---|---|---|---|---|---|---|---|
| NC: data not communicated. | | | | | | | |

### rs488087 SNP frequency in sporadic pancreatic cancers (PC).

The nucleotide sequence encoding the C-terminal domain of the BSDL was amplified by touch-down PCR and sequenced. The analysis was performed on genomic DNA and transcripts from tissue extracts. Analysis of the chromatograms allowed the inventors to highlight the SNP rs488087, c.1719C>T, located in the second repeat in exon 11 sequence (Figure 2). No differences were observed between the data obtained from the analysis of the genomic DNA and those obtained from transcripts (data not shown).

The SNP occurrence was 63.9% in patients with pancreatic cancers (n = 36), whereas it was 25.53% in control subjects (n = 47). The frequency of the T allele was 37.5% (n = 72) in the PC cohort; *versus* 15.96% (n = 94) in the control group (Figure 3). The occurrence and the allele frequency are statistically different between both groups (Chi² test, p = 0.0005 and p = 0.0016, respectively).

When the PC group SNP occurrence (63.9%) was compared to that of the OC group (40.9 %) the difference was also found significant (Chi² test , p = 0.041). Noteworthy the allelic frequency in the PC group was highly significant when compared to the allelic frequency in the rs488087 data bank (UCSC Genome Browser) (37.5 % versus 23.949 %, Chi² test, p = 0.0084).

### rs488087 SNP frequency in non-tumoral diseases of the pancreas (non-TDP).

Sample analysis was performed as described above for pancreatic cancer samples. Sequence analysis showed that only 2/10 of the patients affected with a non-tumoral disease of the pancreas, had the T allele, i.e. 20% (Figure 3). There is no difference between the control group and the non-TDP group (Fisher's Exact Test p = ns.). However, the occurrence of the polymorphism in the non-TDP group was statistically lower than in the PC group (15% vs 63.9%, p = 0.0282).

### rs488087 SNP frequency in non-pancreatic tumoral diseases (OC) and control group (CTL).

When the OC group (40.9 %) SNP occurrence was compared to that of the control group (25.6 %) the difference was not significant (Chi² test, p = 0.1189) (Figure 3). Further no significance was also found when allelic frequency between these two groups (OC group, 23.9 % and CTL group 15.96 %) was examined (Chi² test, p = 0.1809). Noteworthy the allelic frequency was also not significant when the OC group was compared to the allelic frequency in the rs488087 data bank (UCSC Genome Browser) (23.9 %versus 23.949 %, Chi² test, p = 0.9795).

### Germinal statute of the rs488087 SNP

To determine whether the rs488087 SNP has a germinal (constitutional) or somatic (due to malignancy) character, the inventors investigated this polymorphism in genomic DNA extracted from whole blood and/or serum. The results are based on sequence analysis of five patients. These preliminary results show a perfect match of the 5 patients between the gDNA obtained from the tumor, gDNA from the whole blood and serum. This correlation is present in patients with a reference sequence (3 out of 5 patients) and patients with "T" sequence (2 out of 5 patients, one homozygous T/T and one heterozygous C/T) (Figure 4A and Figure 4B, respectively).

### Example 2

### Material and methods

### Droplet Digital PCR (ddPCR)

Digital PCR is a new approach to nucleic acid detection and quantification. This technique is a real innovation in the study of the genetics of cancer. Digital PCR overcomes the difficulties of conventional PCR. With ddPCR, a sample is partitioned so that individual nucleic acid molecules (15 000 droplets of 1nl, limiting dilution of 0 at 1 DNA copy/droplet) within the sample are localized and concentrated within many separate regions. The partitioning of the sample allows one to quantify the molecules according to Poisson Law. Each part will contain "0" or "1" molecules, or a negative or positive reaction, respectively. After PCR amplification, nucleic acids may be quantified by counting the droplet that contain PCR end-product, positive reactions.

The ddPCR reaction contained : 1X ddPCR supermix for probes (Bio-Rad Laboratories, Pleasanton, CA), 100 to 250mM of each probes (comprising a fluorophore quencher pair), and 450 to 900mM of each primers and 33 pg to 50 ng of template DNA in a final volume of 20µL. A total of 22µL of each reaction mixture was prepared to loaded 20 µl without bubbles onto a disposable plastic cartridge (Bio-Rad) with 70µL of droplet generation oil (Bio-Rad) and placed in the droplet generator (Bio-Rad). Indeed, the ddPCR began by partitioning the TaqMan reaction mix containing sample DNA into aqueous droplets in oil via the QX100 Droplet Generator (Bio-Rad); after transfer of droplets to a 96-well PCR plate, a 2-step thermocycling protocol (95°C x 10min; 40 cycles of [94°C x 30s, 54°C x 1 min]; 98°C x 10 min) was carried out in a conventional thermocycleur. The PCR plate was then transferred to the QX100 Droplet Reader for automatic reading of samples in all wells. Data were analyzed with QuantaSoft analysis (version 1.3.2.0) software.

PCR primers used for amplifying segments of exon 11 were :
- BSDL-SNP-Forward primer : 5' -CCCCCACAGGGGAC-3' (SEQ ID NO: 10)
- BSDL-SNP-Reverse primer : 5'- CGGTCTCGGAGTCAC-3' (SEQ ID NO: 11)

Fluorescent reporter probes specific to the C or T allele were :
- SNP-C (specific to the C allele): 5'- hexachloro-6-carboxyfluorescein (HEX)-TGGGGGGCACGGGA-BHQ1-3' (SEQ ID NO: 12)
- SNP-T (specific to the T allele): 5'-6-carboxyfluorescein (6-FAM)-TGGGGGGCACAGGA-BHQ1-3' (SEQ ID NO: 13).

### Results

### Droplet-Digital PCR to detect the rs488087 c.1719C>T SNP.

Droplet-Digital PCR (ddPCR) technology provides an absolute count of target DNA copies per input sample without the need for standard curves determination, making this technique robust for quantification of target DNA. Therefore, the use of ddPCR allowed to count DNA target copies in each DNA samples tested by Sanger sequencing. In these samples the ratio " copy number of SNP sequence C" (quantification of HEX reporter dye) / "copy number SNP sequence T" (quantification of 6-FAM reporter dye) is close to the unit. This confirms that the SNP modification stands for a germinal mutation and not for a somatic one. Figure 5 shows the three possibilities encountered: homozygous patients C/C (reference sequence), homozygous patients T/T and heterozygous patients C/T (cf. Fig. 5, 3. heterozygous patient: ratio C/T = 0.982).

Furthermore, the analyses obtained by Sanger sequencing matched that of ddPCR at 99.12% (i.e 113 samples / 114 showed SNP modification c.1719C>T in ddPCR experiment and corresponding Sanger sequencing).

This percentage shows that these probes are really specific to this SNP and allow simple, rapid and specific detection of this SNP.

### REFERENCES

Amundadottir et al 2009, Nat Genet. September ;41(9) :986-990.
Augé et al. (2003) Circulation. 108:86-91.
Bengtsson-Ellmark et al. (2004) Eur J Hum Genet 12:627-632.
Blackberg et al. (1987) FEBS Lett 217:37-41.
Bruneau et al. (1997) J Biol Chem 272:27353-27361.
Cartwright et al. (2008) Cancer Control. 15:308-313.
Crescence et al. (2012) J Immunol. 189:3386-3396.
Downs et al. (1994) Biochemistry 33:7979-7985.
Griffin and Smith, (2000) Trends in Biotechnology, 18: 77-84.
Jarufe et al. (2005) Surgeon. 3:79-83.
Johansson et al. (2011) J. Biol. Chem. 286 : 34593-605
Lockhart et al. (2005) Gastroenterology. 128:1642-54.
Lombardo D (2001) Biochim Biophys Acta 1533:1-28.
Lombardo D, Guy O (1980) Biochim Biophys Acta 611:147-155
Low et al. 2010 PLoS ONE 5(7) : e11824
Madeyski et al. (1998) Mamm Genome 9:334-338.
Mas et al (1993) Biochem J. 289 : 609-615
Müller-Nordhorn et al. (2006) Digestion. 74:118-125.
Panicot-Dubois et al. (2007) J Clin Invest. 117:3708-3719
Petersen et al., Nat Genet. 2010 March ; 42(3) :224-228
Pliarchopoulou K, Pectasides D (2009) Cancer Treat Rev. 35:431-436.
Raeder et al. (2006) Nat Genet 38:54-62.
Raeder et al. (2007) Diabetes 56:444-449.
Ragvin et al. (2013) Pancreatology. 13 : 29-32.
Rebaï et al. (2005) Arterioscler Thromb Vasc Biol. 25:359-64.
Reuss et al. Int J Oncol, 29 :649-654, 2006
Shu et al., Nucleic Acids Res. Sep 1, 2001; 29(17): e88.
Vesterhus et al. (2008) Diabetes Care 31:1738-1740.
Wang et al. (1995) Biochemistry 34:10639-10644.
Wu et al. Nat Genet 2012 44(1) : 62-66
Xiaoquan et al. Nucleic Acids Res. Nov 15, 2001; 29(22): e116.

## Claims

1. An *in vitro* method for determining a predisposition to, prognosing or diagnosing pancreatic cancer in a subject, comprising detecting the presence or absence of a genetic alteration in exon 11 of the BSDL gene, in a biological sample of said subject.

2. The method of claim 1, wherein the alteration is a substitution, an insertion or a deletion of one or several bases, preferably a single nucleotide polymorphism (SNP).

3. The method of claim 1 or 2, wherein the alteration is the SNP rs488087, or a polymorphic site in linkage disequilibrium with rs488087.

4. The method of any claims 1 to 3, wherein the alteration is the SNP rs488087.

5. The method of claim 4, wherein the identification of a T at polymorphism site rs488087, indicates an increased risk of developing pancreatic cancer.

6. The method of any claims 1 to 5, wherein the biological sample is a tissue, urine, saliva, serum, plasma or whole blood sample.

7. The method of any of claims 1 to 6, wherein the alteration is detected by sequencing, selective hybridization and/or selective amplification, enzyme-based methods or methods relying on differences in the conformation, weight or size of the molecules.

8. The method of any of claims 1 to 7, wherein the pancreatic cancer is an exocrine pancreatic cancer.

9. The method of any of claims 1 to 8, wherein the pancreatic cancer is a pancreatic adenocarcinoma.

10. The method of any of claims 1 to 9, wherein the pancreatic cancer is a sporadic pancreatic cancer.

11. The method of any of claims 1 to 9, wherein the subject has a personal history of diabetes, chronic pancreatitis, preneoplastic pancreatic lesions, hereditary pancreatic cancer or relapses of pancreatic cancer, a family history of pancreatic cancer or pancreatitis, or a particular hereditary condition such as BRCA1/2 mutations, Peutz-Jeghers syndrome, familial atypical mole and melanoma syndrome, von Hippel-Lindau syndrome, Lynch syndrome or Wermer Syndrome.

12. A kit for determining a predisposition to, prognosing or diagnosing pancreatic cancer in a subject, wherein the kit comprises
(i) at least one nucleic acid probe capable of specifically binding or hybridizing to a genetic alteration in exon 11 of the BSDL gene, preferably to the polymorphism site rs488087, and/or
(ii) at least one set of primers suitable for use in amplification reaction, wherein the set of primers amplifies a genetic alteration in exon 11 of the BSDL gene,
and optionally, a leaflet providing guidelines to use such a kit.

13. The kit of claim 12, wherein the kit comprises
a nucleic acid probe comprising, or consisting of, the sequence 5-TGGGGGGCACGGGA-3' (SEQ ID NO: 8), preferably attached to a fluorophore-quencher pair,
and/or
a nucleic acid probe comprising, or consisting of, the sequence 5'-TGGGGGGCACAGGA-3' (SEQ ID NO: 9), preferably attached to a fluorophore-quencher pair.

14. The kit of claim 12 or 13, further comprising one or several reagents for detecting the hybridization of said at least one nucleic acid probe and/or for amplifying and/or detecting said genetic alteration or polymorphism site.

15. The use of the kit of any of claims 12 to 14 for determining a predisposition to, prognosing or diagnosing pancreatic cancer in a subject.
